Europäisches Patentamt

(19) European Patent Office

Office européen des brevets

(11) Publication number : **0 437 281 B1**

(12) **EUROPEAN PATENT SPECIFICATION**

(45) Date of publication of patent specification :
13.04.94 Bulletin 94/15

(51) Int. Cl.[5] : **A61K 37/02, A61K 31/725**

(21) Application number : **91101195.5**

(22) Date of filing : **16.12.85**

(54) Composition comprising acidic fibroblast growth factor (aFGF).

(30) Priority : **24.12.84 US 685923**

(43) Date of publication of application :
**17.07.91 Bulletin 91/29**

(45) Publication of the grant of the patent :
**13.04.94 Bulletin 94/15**

(84) Designated Contracting States :
**CH DE FR GB IT LI NL**

(56) References cited :
US-A- 4 444 760
PROC. NATL. ACAD. SCI. USA, vol. 81, November 1984, pages 6963-6967, Washington, DC, US; G. GOSPODAROWICZ et al.: "Isolation of brain fibroblast growth factor by heparin-Sepharose affinity chromatography: Identity with pituitary fibroblast growth factor"
CHEMICAL ABSTRACTS, vol. 96, no. 19, 10th May 1982, page 168, abstract no. 156286r, Columbus, Ohio, US; P. BUNTROCK et al.: "Stimulation of wound healing, using brain extract with fibroblast growth factor (FGF) activity. II. Histological and morphometric examination of cells and capillaries"

(56) References cited :
JOURNAL OF CELL BIOLOGY, vol. 97, December 1983, pages 1677-1685, New York, NY, US; D. COSPODAROWICZ et al.: "Bovine brain and pituitary fibroblast growth factors: Comparison of their abilities to support the proliferation of human and bovine vascular endothelial cells"
PPOC. NATL. ACAD. SCI. USA, vol. 82, October 1985, pages 6409-6413, Washington, DC, US; K.A. THOMAS et al.: "Pure brain-derived acidic fibroblast growth factor is a potent angiogenic vascular endothelial cell mitogen with sequence homology to interleukin 1"
BIOCHEMISTRY, vol. 24, no. 19, 10th September 1985, pages 4969-4973, Easton, PA, US; R.R. LOBB et al.: "Induction of angiogenesis by bovine brain derived class 1 heparin-binding growth factor"

(60) Publication number of the earlier application in accordance with Art. 76 EPC : **0 186 084**

(73) Proprietor : **MERCK & CO. INC.**
**126, East Lincoln Avenue P.O. Box 2000**
**Rahway New Jersey 07065-0900 (US)**

(72) Inventor : **Thomas, Kenneth A.**
**245 Washington Avenue**
**Chatham, New Jersey 07928 (US)**

(74) Representative : **Abitz, Walter, Dr.-Ing. et al**
**Patentanwälte Abitz & Partner Postfach 86 01 09**
**D-81628 München (DE)**

## Description

The subject invention relates to a composition for the stimulation of growth of vascular endothelial cells comprising aFGF and heparin.

Brain-derived acidic fibroblast growth factor, (aFGF), is an active mitogen for vascular endothelial cells in culture and is useful for growth of such cultures for coverage of polymeric vascular grafts; growth of such cultures on tubular supports for production of blood vessels for implantation; and stimulation or facilitation of blood vessel growth and repair in vivo.

The brain-derived aFGF purification and wound healing activity of this invention were described in US-A-4 444 760 issued April 24, 1984 to Kenneth A. Thomas, Jr. The protein is also described by Thomas et al., Proc. Natl. Acad. Sci., USA 81, 357-361 (1984). The same or similar proteins may also be present in other partially purified extracts from the central nervous system and other organs. See, for example, Maciag et al., Science, 225, 932 (1984).

Although the growth of vascular endothelial cells was accomplished in the past using very high concentrations of fetal calf or adult bovine serum (10-30%) the results were variable, depending on the particular lot of calf serum, and the rate of cell growth was generally slow. With aFGF rapid growth rates are achieved with serum levels from 0 to 2%.

The method of reproducible stimulation of vascular endothelial growth, mediated by pure protein mitogens permits:

1) Covering synthetic polymeric vessels with non-thrombogenic vascular endothelial cells from the host animal, including human, whereby many or all of the clotting problems associated with synthetic vessel grafts are obviated;

2) production of vessels in vitro by growth of host vascular endothelial cells on tubular supports, for implantation back into the same host animal, including human, whereby immunological rejection of the implant will be obviated and the frequent limited supply of good vessels within the patient for transplant will also be obviated; and

3) stimulation or facilitation of blood vessel growth and repair in vivo, whereby the flow of blood to tissues deprived of adequate oxygen and/or other blood borne components is increased.

The brain-derived aFGF used in the novel compositions of this invention is prepared as described in US-A-4 444 760.

The complete amino acid sequence has been determined for aFGF. Sequence determinations of reduced and carboxymethylated protein have revealed two amino termini. The longer sequence, aFGF-1, contains six amino terminal residues not found on the shorter aFGF-2 form. The relative amounts of these two microheterogeneous forms of the mitogen vary from one purification to another but are closely correlated in amount to the abundance of the two bands of protein previously seen by electrophoresis in SDS polyacrylamide gels (Thomas, et al., Proc. Natl. Acad. Sci. USA, 81, 357 (1984)). As expected, the amount of the longer amino terminal sequence correlates with the relative quantity of the higher mass band on the SDS gels. If the length of the polypeptide chain at the amino termini is the only difference between the two microheterogeneous forms observed on the SDS gels, then the mass difference between them is 642 daltons, rather than the previously estimated 200 daltons based on SDS gel migration distances. It is assumed that the amino terminal heterogeneity is the result of limited proteolysis either in vivo or during purification.

The complete amino acid sequence was determined from sequences of the amino termini and overlapping peptides generated by proteolytic cleavages with trypsin (T), Staphylococcus aureus V8 Protease (V8), hydroxylamine (HA) and cyanogen bromide (CN). The carboxyl terminal sequence of the whole protein was confirmed by timed carboxypeptidase A digestion.

In a search of the current Dayhoff protein data bank, aFGF is unique compared to the approximately 2000 protein sequences contained in that list.

The sequence is as follows:

Peptide sequences that were prematurely terminated because they were recognized to begin at one of the two previously determined amino termini are marked with asterisks following the last degradation cycle.

The compositions of the invention comprise heparin in addition to the aFGF discussed above. Those compositions can be used for neovascularization of surface wounds and for internal vascular growth.

The method for the stimulation of vascular endothelial cells comprises treating a sample of the desired vascular endothelial cells in a nutrient medium with aFGF at a concentration of about 1-10 ng/ml. The concentration of heparin is about 10 to 100 µg/ml.

If the vascular endothelial cell growth is conducted in vitro, the presence of a nutrient medium such as

4

Dulbecco's modified Eagle's medium or modification thereof is required and a low concentration of calf or bovine serum such as about 0 to 2% by volume. Preservatives such as a penicillin-streptomycin combination or other broad spectrum antibacterials are also employed.

The novel composition of this invention is useful for the coverage of artificial blood vessels with endothelial cells. Vascular endothelial cells from the patient would be obtained by removal of a small segment of peripheral blood vessel or capillary-containing tissue and the desired cells would be grown in culture in the presence of aFGF and heparin and any other supplemental components that might be required such as serum. After growth of adequate numbers of endothelial cells in culture to cover the synthetic polymeric blood vessel the cells would be plated on the inside surface of the vessel. Prior coating of the artificial vessel either covalently or noncovalently, with either heparin or proteins such as fibrin, collagen, fibronectin or laminin would be performed to enhance attachment of the cells to the artificial vascular surface. The cell-lined artificial vessel would then be surgically implanted into the patient and, being lined with the patients own cells, would be immunologically compatible. The non-thrombogenic endothelial cell lining should decrease the incidence of clot formation on the surface of the artificial vessel and thereby decrease the tendency of vessel blockage or embolism elsewhere.

The novel composition is also useful for the production of artificial vessels. Vascular endothelial cells and smooth muscle cells from the patient would be obtained and grown separately in culture. The endothelial cells would be grown in the presence of the aFGF and heparin as outlined above. The smooth muscle cells would be grown in culture by standard techniques. A tubular mesh matrix of a biocompatible polymer (either a synthetic polymer, with or without a coating of either heparin or specific attachment proteins, or a non-immunogenic biopolymeric material such as surgical suture thread) would be used to support the culture growth of the smooth muscle cells on the exterior side and vascular endothelial cells on the interior surface. Once the endothelial cells form a confluent monolayer on the inside surface and multiple layers of smooth muscle cells cover the outside, the vessel is implanted into the patient.

The novel composition can also be used for the induction of vascular growth. The pure growth factor or the equivalent human protein and heparin would be used to induce and promote the growth of blood vessels in the patient. The mitogen and heparin would be administered along with any necessary stabilizers and enhancers of activity at the site of desired vascular growth. For applications involving neovascularization of surface wounds, such as abrasions or burns, the formulation would be applied directly in a slow release polymer at a rate of about 1-100 ng/day/cm$^2$ of injured surface. For internal vascular growth, the formulation would be released directly into the region to be neovascularized either from implanted slow release polymeric material or from slow release pumps. The release rate in either case is preferably about 100 ng - 10 ug/day/cm$^3$ of injured tissue.

## EXAMPLE

Angiogenic Activity of aFGF Chicken Egg Angiogenesis Bioassay

During the sustained vascular growth, endothelial cells are observed to actively proliferate. Therefore, we tested the ability of the purified mitogen to induce blood vessel growth in the chicken egg chorioallantoic membrane angiogenesis assay. Based on previous reports that crude tumor angiogenesis factor was significantly more active with coadministered heparin, we tested the vascularization response of heparin alone and heparin plus pure aFGF.

Three-day old chicken embryos were removed from their shells and grown in Handiwrap pouches suspended inside paper cups. The tops of the cups were covered with Handiwrap, and the eggs were incubated at 37°C in a tissue culture incubator for 5-6 days. Either 1 μg of pure aFGF in about 30 μl of the HPLC elution solvent (7 mM trifluoroacetic acid/33% acetonitrile) or an identical HPLC solvent control solution were mixed with an equal volume of 2% low-gelling temperature agarose (Miles) dissolved in lactated Ringer's solution (Abbott) containing 10 μg of heparin (from porcine intestinal mucosa; Sigma grade 1). Droplets (60 μl) were allowed to gel on the center of sterile plastic 1.3-cm diameter Thermanox tissue culture coverslips (Miles), and at least part of the volatile acetonitrile evaporated by aeration for 15-30 minutes under a plenum of sterile air in a tissue culture hood. The coverslips were positioned, pellet down, over the chorioallantoic membrane of the eggs and incubated for 3 days. Eggs containing large white focal regions under the coverslips at the end of the assay, presumably formed by inflammatory cells, were discarded. The chorioallantoic membranes were examined microscopically and scored for the proliferation of fine capillaries under the center of cover-slips by observers who did not know the contents of the agarose pellets.

A 10μg dose of heparin per egg was inactive but the same amount of heparin plus 1 μg of aFGF per egg appeared to enhance the growth of small capillaries at the site of application with no sign of inflammation (Table 1). The assay is reproducible, the results being a composite of three separate assays with different samples of aFGF. Control and positive angiogenic responses show the extent of capillary proliferation induced by aFGF. The mitogen is, therefore, a potent angiogenic protein in the presence of heparin.

## TABLE 1

### Angiogenic Activity of aFGF

| Sample contents | Angiogenic response | |
|---|---|---|
| | Negative | Positive |
| Control | 15 | 0 |
| aFGF | 2 | 10 |

These data are a composite of three separate experiments. Using t-distribution statistics, the group of mitogen-stimulated eggs was calculated to be different from the control population with a confidence level of 99.9%.

## Claims

1. A composition for the stimulation of growth of vascular endothelial cells comprising aFGF and heparin.

2. The composition of claim 1 for neovascularization of surface wounds.

3. The composition of claim 1 for internal vascular growth.

## Patentansprüche

1. Eine Zusammensetzung zur Stimulierung des wachstums vaskulärer Endothel-Zellen, umfassend aFGF und Heparin.

2. Die Zusammensetzung nach Anspruch 1 zur Neovaskularisation von Oberflächenwunden.

3. Die Zusammensetzung nach Anspruch 1 zum inneren vaskulären Wachstum.

## Revendications

1. Composition pour la stimulation de la croissance de cellules endothéliales vasculaires comprenant de l'aFGF et de l'héparine.

2. Composition de la revendication 1 pour la néovascularisation de blessures de surface.

3. Composition de la revendication 1 pour la croissance vasculaire interne.